# NEW EUROPEAN PATENT SPECIFICATION

(11) **EP 3 236 918 B2**
(45) Date of publication and mention of the opposition decision: **16.11.2022**
(45) Mention of the grant of the patent: 12.02.2020
(21) Application number: 15813892.5
(22) Date of filing: 23.12.2015
(51) Int. Cl.: A61K 8/37, A61K 8/60, A61K 8/34, A61Q 19/00, A61K 8/81

(54) **USE OF A FATTY ACID ESTER FOR MATTIFYING THE SKIN AND COMPOSITION COMPRISING THIS ESTER**
VERWENDUNG EINES FETTSÄUREESTERS ZUR MATTIERUNG DER HAUT UND ZUSAMMENSETZUNG MIT DIESEM ESTER
UTILISATION D'ESTER D'ACIDE GRAS POUR MATIFIER LA PEAU, ET COMPOSITION COMPRENANT CET ESTER

(30) Priority: 23.12.2014 FR 1463283
(43) Date of publication of application: 01.11.2017
(73) Proprietor: L'OREAL, 75008 Paris (FR)
(72) Inventor: TERRISSE, Isabelle, 94400 Vitry sur Seine (FR); ELGUIDJ, Irène, 92200 Neuilly (FR)
(74) Representative: Casalonga
(86) International application number: PCT/EP2015/081211
(87) International publication number: WO 2016/102695

(56) References cited:
- EP-A1- 1 637 186
- EP-A2- 2 238 967
- WO-A1-98/52536
- WO-A1-2014/191116
- DE-T2-602005 002 810
- FR-A1- 2 962 329
- FR-A1- 2 982 487
- FR-A1- 2 989 896
- US-A1- 2007 269 470
- US-A1- 2009 324 655
- US-A1- 2011 104 220
- US-A1- 2011 274 731
- Anonymous: "GNPD - Anti-Imperfections Moisturiser", , 1 June 2014 (2014-06-01), XP055202558, Retrieved from the Internet: URL:http://www.gnpd.com/sinatra/recordpage /2364421/from_search/nMR9zS7IGW/ [retrieved on 2015-07-15]
- Anonymous: "GNPD - CC Mat Color & Shine Control", , 1 June 2013 (2013-06-01), XP055202543, Retrieved from the Internet: URL:http://www.gnpd.com/sinatra/recordpage /2220272/from_search/74iHHsAExN/ [retrieved on 2015-07-15]
- Anonymous: "GNPD - BB Matte with Signature Shinerase Broad Spectrum SPF 30", , 1 May 2013 (2013-05-01), XP055202550, Retrieved from the Internet: URL:http://www.gnpd.com/sinatra/recordpage /2031929/from_search/74iHHsAExN/ [retrieved on 2015-07-15]
- ANONYMOUS: "Specialised Shampoo", MINTEL, November 2012 (2012-11-01), pages 1 - 2
- ANONYMOUS: "Ultra comfortable face cream", MINTEL, November 2012 (2012-11-01), pages 1 - 8
- ANONYMOUS: "Cutina® MD", HENKEL, 1975, pages 1 - 2

## Description

The present invention relates to the field of caring for and/or making up keratin materials, and in particular the skin.

The present invention relates to the use, especially the cosmetic use, of behenyl behenate, as an agent for mattifying keratin materials, especially oily and/or shiny skin.

It also relates to a composition, especially a cosmetic and/or dermatological composition, comprising, in a physiologically acceptable medium, at least one fatty alcohol comprising at least 20 carbon atoms, behenyl behenate, and at least one thickener.

Another subject of the invention is a process for caring for and/or making up the skin, comprising the topical application of the composition to keratin materials such as the skin.

The invention also relates to a cosmetic process for mattifying the skin and/or for reducing the shininess thereof, comprising the topical application, to said skin, of the aforementioned composition.

The shininess of the skin, which is generally linked to a high level of sebum secretion, is a problem which essentially affects adolescents but which may also occur in adults, especially as a consequence of hyperproduction of androgens or as a consequence of external factors such as pollution. The shininess of the skin may also be linked to sweat resulting from physical activity or climatic conditions (heat, humidity). The shininess of the skin may be due to the combination of the two phenomena (sebum and sweat).

Obtaining a matte effect of the skin is highly desired by users who have combination skin or oily skin, and also for cosmetic compositions that are intended to be used in hot and humid climates. Indeed, the reflections caused by an excess of sebum and/or sweat on the surface of the skin are generally considered esthetically displeasing.

Shiny skin also generally gives rise to a poorer wear property of the makeup, which thus has a tendency to become degraded over the course of the day.

Aside from the use of "sebum-regulating" agents, that is to say agents capable of helping to regulate the activity of the sebaceous glands by an action which may be termed biological, an effective means of rapidly reducing unsightly areas of shininess consists in using "soft focus" fillers. The use of sebum- and perspiration-absorbing fillers is also a means of prolonging the matte finish over time.

It is known practice to use perlite (FR 2881643) or fumed fillers (EP 1 637 186) or else fibers as mattifying agents. However, these fillers may lead to discomfort, especially fluff on the skin and/or a perception of having unclean skin. However, the use of these fillers is generally accompanied by a dry, rough feel and a lack of comfort that is unacceptable to the user.

The need remains for mattifying cosmetic compositions which have good cosmetic properties, and which in particular afford an efficient (strong), immediate and/or long-lasting mattifying effect.

Now, the inventors have demonstrated that fatty acid esters having at least 16 carbon atoms have good properties for mattifying oily and/or shiny skin; indeed, it has been observed that the shininess of the skin is reduced after applying fatty acid ester according to the invention to oily and/or shiny skin. The oily and/or shiny skin treated in this way has a more matte appearance.

The applicant company has discovered that this need was more particularly met by combining one or more alcohols comprising at least 20 carbon atoms, one or more fatty acid esters, said ester comprising at least 24 carbon atoms, and one or more thickeners.

The composition obtained in this way makes it possible to improve the matte finish of the skin in a long-lasting way. The skin thus remains durably mattified.

A subject of the present invention is thus a composition, especially a cosmetic and/or dermatological composition, comprising, in a physiologically acceptable medium:
a) one or more alcohols comprising at least 20 carbon atoms,
b) one or more esters of fatty acids, said fatty acid ester is behenyl behenate, and
c) at least one or more thickeners which are not xanthan gum, wherein the thickeners are chosen from homopolymers and copolymers based on acrylic acid or methacrylic acid which may or may not be salified, homopolymers of 2-acrylamido-2-methylpropanesulfonic acid which may or may not be salified, and/or cellulose polymers, and anionic associative thickeners, preferably acrylic, which may or may not be salified, and
d) at least one C8 -C30 alkyl(poly)glycoside.

The present invention relates to the use, especially the cosmetic use, of behenyl behenate, as a mattifying agent for keratin materials, in particular for the skin and especially in a composition suitable for topical application.

The constituents of the composition according to the invention will now be described in more detail.

### Fatty alcohols comprising at least 20 carbon atoms

The composition of the invention comprises one or more fatty alcohols comprising at least 20 carbon atoms. The fatty alcohols according to the invention comprise at least one aliphatic chain comprising at least 20 carbon atoms. Said aliphatic chain can be linear or branched and saturated or unsaturated. It preferably comprises from 20 to 100 carbon atoms, particularly from 20 to 50 carbon atoms, even more particularly from 20 to 30 carbon atoms. In addition, said fatty alcohol(s) may be hydroxylated. If they are unsaturated, these compounds may comprise one to three conju-gated or unconjugated carbon-carbon double bonds.

These fatty alcohols are not alkoxylated and in particular are not (poly)oxyethylenated and/or (poly)oxypropylenated.

Preferably, the fatty alcohol has the structure **R^{a}-OH,** in which **R^{a}** denotes a saturated or unsaturated, linear or branched, radical comprising from 20 to 50 carbon atoms, preferably from 20 to 30 carbon atoms; **R^{a}** may be substituted with one or more hydroxyl groups, and in particular with one or two hydroxyl groups.

The fatty alcohol may represent a mixture of fatty alcohols, which means that several types of fatty alcohol may coexist, in the form of a mixture, in a commercial product.

Advantageously, the fatty alcohol is solid or pasty at a temperature of 25°C and at atmospheric pressure (10⁵ Pa). Within the context of the present invention, the term *"fatty alcohol which is solid or pasty at 25°C"* is intended to mean a fatty alcohol that has a viscosity, measured with a rheometer (for example an R600 rheometer) at a shear rate of 1 s⁻¹, of greater than or equal to 1 Pa.s. Preferably, the fatty alcohols according to the invention are saturated linear fatty alcohols.

Preferably, they are primary fatty alcohols with a saturated linear chain. Examples that may especially be mentioned include behenyl alcohol, arachidyl alcohol, lignoceryl alcohol, ceryl alcohol, myricyl alcohol and montanyl alcohol. More particularly, behenyl alcohol and/or arachidyl alcohol will be used.

It is also possible to use mixtures of these fatty alcohols with fatty alcohols containing fewer than 20 carbon atoms.

As mixture of fatty alcohols that is particularly preferred in the composition according to the invention, use may be made, for example, of the mixture of fatty alcohols constituted of 76% by weight of behenyl alcohol, 17% by weight of arachidyl alcohol, 1.5% by weight of lignoceryl alcohol, 5% by weight of stearyl alcohol and 0.5% by weight of cetyl alcohol. This mixture is sold under the name Nafol^{®} 1822 C by the company Condea. Other examples that may also be mentioned include the mixture sold under the name Nafol^{®} 2298 by the company Condea, which comprises 98% by weight of behenyl alcohol; the mixture sold under the name Nafol^{®} 20-22 by the company Condea, which comprises 30% by weight of behenyl alcohol, 58% by weight of arachidyl alcohol and 6% by weight of lignoceryl alcohol; or also the mixture sold under the name Nafol^{®} 20+ by the company Condea, which comprises 50% by weight of arachidyl alcohol, 29% by weight of behenyl alcohol, 14% by weight of lignoceryl alcohol and 6% by weight of stearyl alcohol.

The amount of fatty alcohols comprising at least 20 carbon atoms in the composition according to the invention varies especially from 0.1% to 10% by weight, preferably from 0.5% to 10% by weight and better still from 1% to 5% by weight relative to the total weight of the composition.

### Additional fatty acid esters comprising at least 24 carbon atoms

The additional acid esters comprising at least 24 carbon atoms are in particular esters comprising two or three C₁₂-C₃₀ fatty chains.

The total number of carbon atoms of the ester ranges from 30 to 100, preferably from 40 to 80.

They are preferably solid at a temperature lower than or equal to approximately 30°C.

The additional fatty acid esters are especially
A) those corresponding to the formula (I) below:

   R-COO-R' (I)

   in which:
   R and R', which may be identical or different, denote a saturated or unsaturated, linear or branched, hydrocarbon-based group comprising from 14 to 30 carbon atoms and preferably from 14 to 24 carbon atoms.
   The radicals R and R' may be hydroxylated. R and R' are chosen such that the compound of formula (I) is solid at a temperature lower than or equal to approximately 30°C. Preferably, R and R' are identical and denote a C₁₄-C₂₄ alkyl group such as behenyl, stearyl or arachidyl.
   Preferred compounds of formula (I) are, for example, stearyl stearate or arachidyl arachidate.
B) di- or triesters of glycerol and of C₁₂-C₃₀ fatty acids.

Use may especially be made of glyceryl tristearate, glyceryl distearate, glyceryl trihydroxystearate, glyceryl tribehenate and glyceryl dibehenate.

The fatty acid esters according to the invention are generally present in the compositions according to the invention in concentrations ranging from 0.1% to 10% by weight and more preferably from 0.5% to 5% by weight relative to the total weight of the composition.

### Thickeners

As indicated previously, the composition according to the invention comprises at least one or more thickeners, which are not selected in the microbial gums. In a preferred embodiment, the composition of the invention comprises at least one or more thickeners which are not xanthan gum.

The term "thickener" is intended to mean a compound which has a viscosity of greater than 11 000 Pa.s when added at 1% by weight to pure water at pH 7 and at 25°C.
The viscosity is measured in Pa.s for a shear value of 0.001 s⁻¹ using a HAAKE Mars rheometer of cone-plate geometry, made from sand-blasted titanium and with a 35 mm diameter and 2° angle. The measurements are carried out with controlled stress at equilibrium.

The thickeners according to the invention may be of natural or synthetic, organic or inorganic, origin.
The thickeners may be anionic, cationic, amphoteric or nonionic and associative or nonassociative polymers.
It is recalled that *"associative polymers"* are polymers that are capable of reversibly associating with one another or with other molecules.
Their chemical structure more particularly comprises at least one hydrophilic region and at least one hydrophobic region. The term "hydrophobic group" *hydrophobic group"* is intended to mean a radical or polymer with a saturated or unsaturated, linear or branched, hydrocarbon-based chain, comprising at least 10 carbon atoms, preferably from 10 to 30 carbon atoms, in particular from 12 to 30 carbon atoms and more preferably from 18 to 30 carbon atoms.
Preferably, the hydrocarbon-based group is derived from a monofunctional compound. By way of example, the hydrophobic group may be derived from a fatty alcohol such as stearyl alcohol, dodecyl alcohol or decyl alcohol. It may also denote a hydrocarbon-based polymer, for instance polybutadiene.

Nonassociative thickening polymers that may be mentioned include nonassociative thickening polymers bearing sugar units.
Within the context of the present invention, the term "sugar unit" is intended to mean a unit derived from a carbohydrate of formula Cₙ(H₂O)ₙ₋₁ or (CH₂O)ₙ, which may be optionally modified by substitution and/or by oxidation and/or by dehydration.
The sugar units that may be included in the composition of the thickening polymers of the invention are preferably derived from the following sugars:
▪ glucose; galactose; arabinose; rhamnose; mannose; xylose; fucose; anhydrogalactose; galacturonic acid; glucuronic acid; mannuronic acid; galactose sulfate; anhydrogalactose sulfate and fructose.
Thickening polymers of the invention that may especially be mentioned include native gums such as:
a) tree or shrub exudates,
b) gums resulting from algae, including:
   ▪ agar (polymer derived from galactose and anhydrogalactose);
   ▪ alginates (polymers of mannuronic acid and glucuronic acid) and salts thereof;
   ▪ carrageenans and furcellerans (polymers of galactose sulfate and anhydrogalactose sulfate);
c) gums derived from seeds or tubers, including:
   ▪ guar gum (polymer of mannose and galactose);
   ▪ locust bean gum (polymer of mannose and galactose);
   ▪ fenugreek gum (polymer of mannose and galactose);
   ▪ tamarind gum (polymer of galactose, xylose and glucose);
   ▪ konjac gum (polymer of glucose and mannose);
d) plant extracts, including:
   ▪ cellulose (glucose polymer);
   ▪ starch (glucose polymer) and
   ▪ inulin.
These polymers can be physically or chemically modified. As physical treatment, mention may especially be made of the temperature.

As chemical treatments, mention may be made of esterification, etherification, amidation or oxidation reactions. These treatments can lead to polymers that may especially be nonionic, anionic or amphoteric.
Preferably, these chemical or physical treatments are applied to guar gums, locust bean gums, starches and celluloses. The nonionic guar gums that may be used according to the invention may be modified with C₁-C₆ (poly)hydroxyalkyl groups.
Among the C₁-C₆ (poly)hydroxyalkyl groups, mention may be made, as example, of the hydroxymethyl, hydroxyethyl, hydroxypropyl and hydroxybutyl groups.
These guar gums are well known in the prior art and can be prepared, for example, by reacting the corresponding alkene oxides, for instance propylene oxides, with the guar gum so as to obtain a guar gum modified with hydroxypropyl groups. The degree of hydroxyalkylation preferably varies from 0.4 to 1.2 and corresponds to the number of alkylene oxide molecules consumed, divided by the number of free hydroxyl functional groups present on the guar gum.
Such nonionic guar gums optionally modified with hydroxyalkyl groups are sold, for example, under the trade names Jaguar HP8, Jaguar HP60, Jaguar HP105 and Jaguar HP120 by the company Rhodia Chimie.
The botanical origin of the starch molecules used in the present invention may be cereals or tubers. Thus, the starches are chosen, for example, from corn starch, rice starch, cassava starch, barley starch, potato starch, wheat starch, sorghum starch and pea starch.
The starches may be chemically or physically modified, especially by one or more of the following reactions: pregelatinization, oxidation, crosslinking, esterification, etherification, amidation, heat treatments.
Use will preferably be made of distarch phosphates or compounds rich in distarch phosphate, such as the product provided under the references Prejel VA-70-T AGGL (gelatinized hydroxypropyl cassava distarch phosphate), Prejel TK1 (gelatinized cassava distarch phosphate) or Prejel 200 (gelatinized acetyl cassava distarch phosphate) by the company Avebe, or Structure Zea from National Starch (gelatinized corn distarch phosphate).
According to the invention, amphoteric starches may also be used, these amphoteric starches comprising one or more anionic groups and one or more cationic groups. The anionic and cationic groups can be bonded to the same reactive site of the starch molecule or to different reactive sites; they are preferably bonded to the same reactive site. The anionic groups may be of carboxylic, phosphate or sulfate type, preferably carboxylic type. The cationic groups can be of primary, secondary, tertiary or quaternary amine type.
The starch molecules may be derived from any plant source of starch, especially such as corn, potato, oats, rice, tapioca, sorghum, barley or wheat. It is also possible to use the hydrolysates of the starches mentioned above. The starch is preferably derived from potato.
The nonassociative thickening polymers of the invention may be cellulose-based polymers not comprising a C₁₀-C₃₀ fatty chain in their structure.
According to the invention, the term *"cellulose-based"* polymer is intended to mean any polysaccharide compound having in its structure sequences of glucose residues linked together via β-1,4 bonds; in addition to unsubstituted celluloses, the cellulose derivatives may be anionic, cationic, amphoteric or nonionic.
Thus, the cellulose-based polymers of the invention may be chosen from unsubstituted celluloses, including those in a microcrystalline form, and cellulose ethers.
Among these cellulose-based polymers, cellulose ethers, cellulose esters and cellulose ester ethers are distinguished. Among the cellulose esters are inorganic cellulose esters (cellulose nitrates, sulfates, phosphates, etc.), organic cellulose esters (cellulose monoacetates, triacetates, amidopropionates, acetate butyrates, acetate propionates, acetate trimellitates, etc.) and mixed organic/inorganic cellulose esters, such as cellulose acetate butyrate sulfates and cellulose acetate propionate sulfates. Among the cellulose ester ethers, mention may be made of hydroxypropylmethylcellulose phthalates and ethylcellulose sulfates.
Among the nonionic cellulose ethers without a C₁₀-C₃₀ fatty chain, i.e. which are *"nonassociative",* mention may be made of (C₁-C₄)alkylcelluloses, such as methylcelluloses and ethylcelluloses (for example, Ethocel standard 100 Premium from Dow Chemical); (poly)hydroxy(C₁-C₄)alkylcelluloses, such as hydroxymethylcelluloses, hydroxyethylcelluloses (for example, Natrosol 250 HHR provided by Aqualon) and hydroxypropylcelluloses (for example, Klucel EF from Aqualon); mixed (poly)hydroxy(C₁-C₄)alkyl(C₁-C₄)alkylcelluloses, such as hydroxypropylmethylcelluloses (for example, Methocel E4M from Dow Chemical), hydroxyethylmethylcelluloses, hydroxyethylethylcelluloses (for example, Bermocoll E 481 FQ from Akzo Nobel) and hydroxybutylmethylcelluloses.
Among the anionic cellulose ethers without a fatty chain, mention may be made of (poly)carboxy(C₁-C₄)alkylcelluloses and salts thereof. By way of example, mention may be made of carboxymethylcelluloses, carboxymethylmethylcelluloses (for example, Blanose 7M from the company Aqualon) and carboxymethylhydroxyethylcelluloses, and the sodium salts thereof.
Among the cationic cellulose ethers without a fatty chain, mention may be made of cationic cellulose derivatives such as cellulose copolymers or cellulose derivatives grafted with a water-soluble quaternary ammonium monomer, and described in particular in patent US 4 131 576, such as (poly)hydroxy(C₁-C₄)alkyl celluloses, for instance hydroxymethyl-, hydroxyethyl- or hydroxypropylcelluloses, grafted especially with a methacryloyloxyethyltrimethylammonium, methacrylamidopropyltrimethylammonium or dimethyldiallylammonium salt. The commercial products corresponding to this definition are more particularly the products sold under the names Celquat^{®} L 200 and Celquat^{®} H 100 by the company National Starch. Among the nonassociative thickening polymers not bearing sugar units that may be used, mention may be made of crosslinked acrylic acid or methacrylic acid homopolymers or copolymers, crosslinked 2-acrylamido-2-methylpropanesulfonic acid homopolymers and crosslinked acrylamide copolymers thereof, ammonium acrylate homopolymers, or copolymers of ammonium acrylate and of acrylamide, alone or as mixtures.
A first family of nonassociative thickening polymers that is suitable is represented by optionally salified crosslinked acrylic acid homopolymers.
Among the homopolymers of this type, mention may be made of those crosslinked with an allyl alcohol ether of the sugar series, for instance the products sold under the names Carbopol 980, 981, 954, 2984 and 5984 by the company Noveon or the products sold under the names Synthalen M and Synthalen K by the company 3 VSA.
The nonassociative thickening polymers may also be crosslinked (meth)acrylic acid copolymers, such as the polymer sold under the name Aqua SF1 by the company Noveon.
The nonassociative thickening polymers may be chosen from crosslinked 2-acrylamido-2-methylpropanesulfonic acid homopolymers and the salts thereof. Among the partially or totally neutralized crosslinked 2-acrylamido-2-methylpropanesulfonic acid polymers, mention may be made in particular of Simulgel 800 from SEPPIC.
The composition may similarly comprise, as nonassociative thickening polymers, ammonium acrylate homopolymers or copolymers of ammonium acrylate and of acrylamide.
Examples of ammonium acrylate homopolymers that may be mentioned include the product sold under the name Microsap PAS 5193 by the company Hoechst. Among the copolymers of ammonium acrylate and of acrylamide, mention may be made of the product sold under the name Bozepol C Nouveau or the product PAS 5193 sold by the company Hoechst. Reference may be made especially to the documents FR 2 416 723, US 2 798 053 and US 2 923 692 as regards the description and the preparation of such compounds.

Among the thickening polymers, mention may also be made of the associative polymers that are well known to a person skilled in the art and especially of nonionic, anionic, cationic or amphoteric nature.

Among the associative polymers of anionic type, mention may be made of:
- (a) those comprising at least one hydrophilic unit and at least one fatty-chain allyl ether unit, more particularly those whose hydrophilic unit is constituted of an ethylenic unsaturated anionic monomer, more particularly still by a vinylcarboxylic acid and most particularly by an acrylic acid or a methacrylic acid or the mixtures thereof.
Among these anionic associative polymers, those that are particularly preferred according to the invention are polymers formed from 20% to 60% by weight of acrylic acid and/or of methacrylic acid, from 5% to 60% by weight of lower alkyl (meth)acrylates, from 2% to 50% by weight of fatty-chain allyl ether, and from 0 to 1% by weight of a crosslinking agent which is a well-known copolymerizable unsaturated polyethylenic monomer, for instance diallyl phthalate, allyl (meth)acrylate, divinylbenzene, (poly)ethylene glycol dimethacrylate and methylenebisacrylamide.

Among the latter polymers, those most particularly preferred are crosslinked terpolymers of methacrylic acid, of ethyl acrylate and of polyethylene glycol (10 EO) stearyl alcohol ether (Steareth-10), especially those sold by the company BASF under the names Salcare SC80^{®} and Salcare SC90^{®}, which are 30% aqueous emulsions of a crosslinked terpolymer of methacrylic acid, of ethyl acrylate and of steareth-10 allyl ether (40/50/10).
- (b) those comprising i) at least one hydrophilic unit of unsaturated olefinic carboxylic acid type, and ii) at least one hydrophobic unit of (C₁₀-C₃₀) alkyl ester of unsaturated carboxylic acid type.
(C₁₀-C₃₀) Alkyl esters of unsaturated carboxylic acids that are useful in the invention comprise, for example, lauryl acrylate, stearyl acrylate, decyl acrylate, isodecyl acrylate and dodecyl acrylate, and the corresponding methacrylates, lauryl methacrylate, stearyl methacrylate, decyl methacrylate, isodecyl methacrylate and dodecyl methacrylate.
Anionic polymers of this type are described and prepared, for example, according to patents US 3 915 921 and US 4 509 949.
Among anionic associative polymers of this type, use will more particularly be made of those constituted of 95% to 60% by weight of acrylic acid (hydrophilic unit), 4% to 40% by weight of C₁₀-C₃₀ alkyl acrylate (hydrophobic unit) and 0% to 6% by weight of crosslinking polymerizable monomer, or else of those constituted of 98% to 96% by weight of acrylic acid (hydrophilic unit), 1% to 4% by weight of C₁₀-C₃₀ alkyl acrylate (hydrophobic unit) and 0.1% to 0.6% by weight of crosslinking polymerizable monomer such as those described above.
Among said polymers above, those most particularly preferred according to the present invention are the products sold by the company Goodrich under the trade names Pemulen TR1^{®}, Pemulen TR2^{®} and Carbopol 1382^{®}, and even more preferably Pemulen TR1^{®}, and the product sold by the company SEPPIC under the name Coatex SX^{®}.

Mention may also be made of the acrylic acid/lauryl methacrylate/vinylpyrrolidone terpolymer sold under the name Acrylidone LM by the company ISP.
- (c) maleic anhydride/C₃₀-C₃₈ α-olefin/alkyl maleate terpolymers, such as the product (maleic anhydride/C₃₀-C₃₈ α-olefin/isopropyl maleate copolymers) sold under the name Performa V 1608^{®} by the company Newphase Technologies.
- (d) acrylic terpolymers comprising:
   i) approximately 20% to 70% by weight of an α,β-monoethylenically unsaturated carboxylic acid **[A],**
   ii) approximately 20% to 80% by weight of an α,β-monoethylenically unsaturated non-surface-active monomer other than **[A],**
   iii) approximately 0.5% to 60% by weight of a nonionic monourethane which is the product of reaction of a monohydric surfactant with a monoethylenically unsaturated monoisocyanate,
   such as those described in patent application EP-A-0 173 109 and more particularly the terpolymer described in Example 3, namely a methacrylic acid/methyl acrylate/ethoxylated (40 EO) behenyl alcohol dimethyl-meta-isopropenylbenzyl isocyanate terpolymer, as a 25% aqueous dispersion.
- ***(e)*** copolymers comprising among their monomers an α,β-monoethylenically unsaturated carboxylic acid and an ester of an α,β-monoethylenically unsaturated carboxylic acid and of an oxyalkylenated fatty alcohol.
Preferably, these compounds also comprise as monomer an ester of an α,β-monoethylenically unsaturated carboxylic acid and of a C₁-C₄ alcohol.
By way of example of this type of compound, mention may be made of Aculyn 22^{®} and Aculyn 88^{®}, sold by the company Rohm and Haas, which are methacrylic acid/ethyl acrylate/oxyalkylenated stearyl methacrylate terpolymers, or else of Aculyn 28^{®}, sold by the company Rohm and Haas, which is a methacrylic acid/ethyl acrylate/oxyalkylenated behenyl methacrylate terpolymer. Mention will also be made of the products sold by the company Lubrizol under the commercial references Carbopol Ultrez 20 and Carbopol Ultrez 21, which are acrylic polymers (acrylates/C10-30 alkyl acrylate crosspolymer), and Novethix L-10, which is an acrylates/beheneth-25 methacrylate copolymer.
- ***(f)*** associative polymers comprising at least one ethylenically unsaturated monomer bearing a sulfonic group, in free or partially or totally neutralized form, and comprising at least one hydrophobic part. These polymers may be crosslinked or noncrosslinked. They are preferably crosslinked.
The ethylenically unsaturated monomers bearing a sulfonic group are especially chosen from vinylsulfonic acid, styrenesulfonic acid, (meth)acrylamido(C₁-C₂₂)alkylsulfonic acids, *N*-(C₁-C₂₂)alkyl(meth)acrylamido(C₁-C₂₂)alkylsulfonic acids such as undecylacrylamidomethanesulfonic acid, and also the partially or totally neutralized forms thereof.

More preferably, use will be made of (meth)acrylamido(C₁-C₂₂)alkylsulfonic acids, such as, for example, acrylamidomethanesulfonic acid, acrylamidoethanesulfonic acid, acrylamidopropanesulfonic acid, 2-acrylamido-2-methylpropanesulfonic acid, methacrylamido-2-methylpropanesulfonic acid, 2-acrylamido-n-butanesulfonic acid, 2-acrylamido-2,4,4-trimethylpentanesulfonic acid, 2-methacrylamidododecylsulfonic acid or 2-acrylamido-2,6-dimethyl-3-heptanesulfonic acid, and also the partially or totally neutralized forms thereof. 2-Acrylamido-2-methylpropanesulfonic acid (AMPS), and also the partially or totally neutralized forms thereof, will more particularly be used.
The polymers of this family may be chosen especially from random amphiphilic AMPS polymers modified by reaction with a C₆-C₂₂ mono-n-alkyl-amine or di-n-alkyl-amine, and such as those described in patent application WO 00/31154 (forming an integral part of the content of the description). These polymers may also contain other ethylenically unsaturated hydrophilic monomers chosen, for example, from (meth)acrylic acids, the β-substituted alkyl derivatives thereof or the esters thereof obtained with monoalcohols or mono- or polyalkylene glycols, (meth)acrylamides, vinylpyrrolidone, maleic anhydride, itaconic acid or maleic acid, or the mixtures of these compounds.
The preferred polymers of this family are chosen from amphiphilic copolymers of AMPS and of at least one ethylenically unsaturated hydrophobic monomer. These same copolymers may also contain one or more ethylenically unsaturated monomers not comprising a fatty chain, such as (meth)acrylic acids, the β-substituted alkyl derivatives thereof or the esters thereof obtained with monoalcohols or mono- or polyalkylene glycols, (meth)acrylamides, vinylpyrrolidone, maleic anhydride, itaconic acid or maleic acid, or the mixtures of these compounds.
These copolymers are described especially in patent application EP-A-750 899, in patent US 5 089 578 and in the following Yotaro Morishima publications:
∘ "Self-assembling amphiphilic polyelectrolytes and their nanostructures - Chinese Journal of Polymer Science, Vol. 18, No. 40 (2000), 323-336";
∘ "Micelle formation of random copolymers of sodium 2-(acrylamido)-2-methylpropanesulfonate and a non-ionic surfactant macromonomer in water as studied by fluorescence and dynamic light scattering - Macromolecules, Vol. 33, No. 10 (2000), 3694-3704";
∘ "Solution properties of micelle networks formed by non-ionic moieties covalently bound to an polyelectrolyte: salt effects on rheological behavior - Langmuir, Vol. 16, No. 12 (2000), 5324-5332";
∘ "Stimuli responsive amphiphilic copolymers of sodium 2-(acrylamido)-2-methylpropanesulfonate and associative macromonomers - Polym. Preprint, Div. Polym. Chem., 40(2) (1999), 220-221".
Among these polymers, mention may be made of:
- crosslinked or noncrosslinked, neutralized or nonneutralized, copolymers, comprising from 15% to 60% by weight of AMPS units and from 40% to 85% by weight of (C₈-C₁₆)alkyl(meth)acrylamide or (C₈-C₁₆)alkyl (meth)acrylate units relative to the polymer, such as those described in application EP-A 750 899;
- terpolymers comprising from 10 mol% to 90 mol% of acrylamide units, from 0.1 mol% to 10 mol% of AMPS units and from 5 mol% to 80 mol% of n-(C₆-C₁₈)alkylacrylamide units, such as those described in patent US-5 089 578.
- All these anionic associative polymers may be salified.
Mention may also be made of copolymers of totally neutralized AMPS and of dodecyl methacrylate, and also crosslinked and noncrosslinked copolymers of AMPS and of n-dodecylmethacrylamide, such as those described in the Morishima papers mentioned above.

Among the cationic associative polymers, mention may be made of:
- ***(I)*** cationic associative polyurethanes;
- ***(II)*** the compound sold by the company Noveon under the name Aqua CC and which corresponds to the INCI name Polyacrylate-1 Crosspolymer. Polyacrylate-1 Crosspolymer is the product of the polymerization of a monomer mixture comprising:
   ∘ a di(C₁-C₄ alkyl)amino(C₁-C₆ alkyl) methacrylate,
   ∘ one or more C₁-C₃₀ alkyl esters of (meth)acrylic acid,
   ∘ a polyethoxylated C₁₀-C₃₀ alkyl methacrylate (20-25 mol of ethylene oxide units),
   ∘ a 30/5 polyethylene glycol/polypropylene glycol allyl ether,
   ∘ a hydroxy(C₂-C₆ alkyl) methacrylate, and
   ∘ an ethylene glycol dimethacrylate.
- ***(III)*** quaternized (poly)hydroxyethylcelluloses modified with groups comprising at least one fatty chain, such as alkyl, arylalkyl or alkylaryl groups comprising at least 8 carbon atoms, or mixtures thereof. The alkyl radicals borne by the above quaternized celluloses or hydroxyethylcelluloses preferably comprise from 8 to 30 carbon atoms. The aryl radicals preferably denote the phenyl, benzyl, naphthyl or anthryl groups. Examples of quaternized alkylhydroxyethylcelluloses containing C₈-C₃₀ fatty chains that may be indicated include the products Quatrisoft LM 200^{®}, Quatrisoft LM-X 529-18-A^{®}, Quatrisoft LM-X 529-18-B^{®} (C₁₂ alkyl) and Quatrisoft LM-X 529-8^{®} (C₁₈ alkyl) sold by the company Aqualon, the products Crodacel QM^{®}, Crodacel QL^{®} (C₁₂ alkyl) and Crodacel QS^{®} (C₁₈ alkyl) sold by the company Croda and the product Softcat SL 100^{®} sold by the company Aqualon.
- ***(IV)*** cationic polyvinyllactam polymers.
Such polymers are described, for example, in patent application WO-00/68282.

As cationic poly(vinyllactam) polymers according to the invention, vinylpyrrolidone/dimethylaminopropylmethacrylamide/dodecyldimethyl methacrylamidopropylammoniumtosylateterpolymers, vinylpyrrolidone/dimethylaminopropylmethacrylamide/cocoyldimethyl methacrylamidopropylammonium tosylate terpolymers, vinylpyrrolidone/dimethylaminopropylmethacrylamide/lauryldimethyl methacrylamidopropylammonium tosylate or chloride terpolymers are used in particular.
The amphoteric associative polymers are preferably chosen from those comprising at least one noncyclic cationic unit. Even more particularly, those prepared from or comprising 1 mol% to 20 mol%, preferably 1.5 mol% to 15 mol% and even more particularly 1.5 mol% to 6 mol% of fatty-chain monomer relative to the total number of moles of monomers are preferred.
Amphoteric associative polymers according to the invention are described and prepared, for example, in patent application WO 98/44012.
Among the amphoteric associative polymers according to the invention, the ones that are preferred are acrylic acid/(meth)acrylamidopropyl-trimethylammonium chloride/stearyl methacrylate terpolymers.

The associative polymers of nonionic type that may be used according to the invention are preferably chosen from:
- (a) copolymers of vinylpyrrolidone and of hydrophobic fatty-chain monomers, examples of which that may be mentioned include:
   - the products Antaron V216^{®} or Ganex V216^{®} (vinylpyrrolidone/hexadecene copolymer) sold by the company I.S.P.
   - the products Antaron V220^{®} or Ganex V220^{®} (vinylpyrrolidone/eicosene copolymer) sold by the company I.S.P.
- (b) copolymers of C₁-C₆ alkyl methacrylates or acrylates and of amphiphilic monomers comprising at least one fatty chain, such as, for example, the oxyethylenated stearyl acrylate/methyl acrylate copolymer sold by the company Goldschmidt under the name Antil 208^{®}.
- (c) copolymers of hydrophilic methacrylates or acrylates and of hydrophobic monomers comprising at least one fatty chain, for instance the polyethylene glycol methacrylate/lauryl methacrylate copolymer.
- (d) polyurethane polyethers comprising in their chain both hydrophilic blocks usually of polyoxyethylenated nature and hydrophobic blocks, which may be aliphatic sequences alone and/or cycloaliphatic and/or aromatic sequences.
- (e) polymers with an aminoplast ether backbone containing at least one fatty chain, such as the Pure Thix^{®} compounds provided by the company Sud-Chemie.
- (f) celluloses or the derivatives thereof, modified with groups comprising at least one fatty chain, such as alkyl, arylalkyl or alkylaryl groups or the mixtures thereof in which the alkyl groups are C₈-C₂₂ alkyl groups, and in particular:
   * nonionic alkylhydroxyethylcelluloses, such as the products Natrosol Plus Grade 330 CS and Polysurf 67 (C₁₆ alkyl) sold by the company Aqualon;
   * nonionic nonoxynylhydroxyethylcelluloses, such as the product Amercell HM-1500 sold by the company Amerchol;
   * nonionic alkylcelluloses, such as the product Bermocoll EHM 100 sold by the company Berol Nobel;
- (g) associative guar derivatives, for instance hydroxypropyl guars modified with a fatty chain, such as the product Esaflor HM 22 (modified with a C₂₂ alkyl chain) sold by the company Lamberti; the product Miracare XC 95-3 (modified with a C₁₄ alkyl chain) and the product RE 205-146 (modified with a C₂₀ alkyl chain) sold by Rhodia Chimie.

Preferably, the polyurethane polyethers comprise at least two lipophilic hydrocarbon-based chains containing from 6 to 30 carbon atoms, separated by a hydrophilic block, the hydrocarbon-based chains possibly being pendent chains or chains at the end of a hydrophilic block. In particular, it is possible for one or more pendent chains to be provided. In addition, the polymer can comprise a hydrocarbon-based chain at one end or at both ends of a hydrophilic block.
The polyurethane polyethers can be multiblock, in particular in triblock form. The hydrophobic blocks can be at each end of the chain (for example: triblock copolymer having a hydrophilic central block) or distributed both at the ends and in the chain (for example, multiblock copolymer). These same polymers can also be grafted polymers or star polymers. The nonionic fatty-chain polyurethane polyethers can be triblock copolymers, the hydrophilic block of which is a polyoxyethylene chain comprising from 50 to 1000 oxyethylene groups. The nonionic polyurethane polyethers comprise a urethane bond between the hydrophilic blocks, hence the origin of the name. By extension, also included among the nonionic fatty-chain polyurethane polyethers are those in which the hydrophilic blocks are linked to the lipophilic blocks via other chemical bonds.
As examples of nonionic fatty-chain polyurethane polyethers that may be used in the invention, it is also possible to use Rheolate 205^{®} containing a urea functional group, sold by the company Rheox, or Rheolate@ 208, 204 or 212, and also Acrysol RM 184^{®}.
Mention may also be made of the product Elfacos T210^{®} containing a C₁₂₋₁₄ alkyl chain, and the product Elfacos T212^{®} containing a C₁₈ alkyl chain, from Akzo.

The product DW 1206B^{®} from Rohm and Haas containing a C₂₀ alkyl chain and a urethane bond, provided at a solids content of 20% in water, may also be used.
Use may also be made of solutions or dispersions of these polymers, in particular in water or in an aqueous/alcoholic medium. Examples of such polymers that may be mentioned are Rheolate@ 255, Rheolate@ 278 and Rheolate@ 244 sold by the company Rheox. Use may also be made of the products DW1206F and DW1206J provided by the company Rohm and Haas. The polyurethane polyethers which can be used according to the invention are in particular those described in the paper by G. Fonnum, J. Bakke and Fk. Hansen - Colloid Polym. Sci., 271, 380-389 (1993).
It is even more particularly preferred to use a polyurethane polyether that may be obtained by polycondensation of at least three compounds comprising (i) at least one polyethylene glycol comprising from 150 to 180 mol of ethylene oxide, (ii) stearyl alcohol or decyl alcohol, and (iii) at least one diisocyanate.
Such polyurethane polyethers are sold in particular by the company Rohm and Haas under the names Aculyn 46^{®} and Aculyn 44^{®} [Aculyn 46^{®} is a polycondensate of polyethylene glycol containing 150 or 180 mol of ethylene oxide, of stearyl alcohol and of methylenebis(4-cyclohexyl isocyanate) (SMDI), at 15% by weight in a matrix of maltodextrin (4%) and water (81%); Aculyn 44^{®} is a polycondensate of polyethylene glycol containing 150 or 180 mol of ethylene oxide, of decyl alcohol and of methylenebis(4-cyclohexyl isocyanate) (SMDI), at 35% by weight in a mixture of propylene glycol (39%) and water (26%)].

In a first variant of the invention, the thickening polymer(s) are chosen from homopolymers and copolymers based on acrylic acid or methacrylic acid which may or may not be salified, homopolymers of 2-acrylamido-2-methylpropanesulfonic acid which may or may not be salified, and/or cellulose polymers.
In a second variant of the invention, the thickening polymer(s) are chosen from anionic associative thickeners, preferably acrylic, which may or may not be salified. The polymers sold under the names Pemulen TR1 or TR2 by the company Lubrizol, the INCI name of which is Acrylates/C10-30 Alkyl Acrylate Crosspolymer, or Aculyn 22 or 88 by the company Rohm and Haas, with the INCI name Acrylates/steareth-20 methacrylate copolymer, or else Aculyn 28 from Rohm and Haas with the INCI name Acrylates/beheneth-25 methacrylate copolymer, will especially be chosen.

The mineral thickeners may be chosen from optionally modified clays, optionally modified silicas or the mixtures thereof.

The optionally modified clays are, for example, smectites such as saponites, hectorites, montmorillonites, bentonites or beidellite and in particular synthetic hectorites (also known as laponites), for instance the products sold by the company Laporte under the names Laponite XLG, Laponite RD and Laponite RDS (these products are sodium magnesium silicates and in particular sodium lithium magnesium silicates); bentonites, for instance the product sold under the name Bentone HC by the company Rheox; magnesium aluminum silicates, which are especially hydrated, for instance the products sold by the company Vanderbilt Company under the names Veegum Ultra, Veegum HS and Veegum DGT, or else calcium silicates and especially the product in synthetic form sold by the company Fitz chem corporation under the name Micro-cel C.

The optionally modified silicas are for example fumed silica optionally subjected to a hydrophobic surface treatment, the particle size of which is less than 1 µm. It is possible especially to replace silanol groups with hydrophobic groups: a hydrophobic silica is then obtained. The hydrophobic groups may be:
- trimethylsiloxyl groups, which are obtained especially by treating fumed silica in the presence of hexamethyldisilazane. Silicas thus treated are known as "Silica Silylate" according to the CTFA (6th edition, 1995). They are sold, for example, under the references Aerosil R812^{®} by the company Degussa and Cab-O-Sil TS-530^{®} by the company Cabot,
- dimethylsilyloxyl or polydimethylsiloxane groups, which are obtained especially by treating fumed silica in the presence of polydimethylsiloxane or dimethyldichlorosilane. Silicas thus treated are known as "Silica dimethyl silylate" according to the CTFA (6th edition, 1995). They are sold, for example, under the references Aerosil R972^{®} and Aerosil R974^{®} by the company Degussa and Cab-O-Sil TS-610^{®} and Cab-O-Sil TS-720^{®} by the company Cabot.
The hydrophobic fumed silica in particular has a particle size that may be nanometric to micrometric, for example ranging from approximately 5 to 200 nm.

Preferably, the mineral thickeners are chosen from lipophilic clays, in particular modified hectorites; hydrophobic-treated fumed silica; hydrophobic silica aerogels, or the mixtures thereof.

In a particular embodiment, the composition according to the invention may further comprise at least one or more additional thickeners selected among those listed above, as well as in the microbial gums, such as xanthan gum.

The thickeners according to the invention are generally present in the composition according to the invention in a content ranging from 0.01% to 20% by weight, preferably ranging from 0.1% to 20% by weight and most preferably ranging from 0.5% to 10% by weight relative to the total weight of the composition.

The composition according to the invention may be cosmetic and/or dermatological, preferably cosmetic.

The composition according to the invention is generally suitable for topical application to the skin and thus generally comprises a physiologically acceptable medium, i.e. a medium that is compatible with the skin and/or its integuments. It is preferably a cosmetically acceptable medium, i.e. a medium which has a pleasant color, odor and feel and which does not cause any unacceptable discomfort (stinging, tautness or redness) liable to discourage the consumer from using this composition.

The composition according to the invention may be in any galenical form conventionally used for topical application and especially in the form of dispersions of gel or lotion type, of emulsions of liquid or semi-liquid consistency of the milk type, obtained by dispersing a fatty phase in an aqueous phase (O/W) or vice versa (W/O), or of suspensions or emulsions of soft, semisolid or solid consistency of the cream or gel type, or alternatively of multiple emulsions (W/O/W or O/W/O), or of microemulsions. As a variant, the composition according to the invention may be in the form of sticks. These compositions are prepared according to the usual methods.
In addition, the compositions used according to the invention can be more or less fluid and can have the appearance of a white or colored cream, of an ointment, of a milk, of a lotion, of a serum, of a paste or of a foam. They may also be in solid form, for example in stick form.
Preferably, the compositions are liquid.
When the composition used according to the invention comprises an oily phase, it preferably contains at least one oil. It may also contain other fatty substances.

As oils that may be used in the composition of the invention, examples that may be mentioned include:
- hydrocarbon-based oils of animal origin, such as perhydrosqualene;
- hydrocarbon-based oils of plant origin, such as liquid triglycerides of fatty acids containing from 4 to 10 carbon atoms, for instance heptanoic or octanoic acid triglycerides, or else, for example, sunflower oil, corn oil, soybean oil, marrow oil, grapeseed oil, sesame seed oil, hazelnut oil, apricot oil, macadamia oil, arara oil, castor oil, avocado oil, caprylic/capric acid triglycerides, for instance those sold by the company Stéarineries Dubois or those sold under the names Miglyol 810, 812 and 818 by the company Dynamit Nobel, jojoba oil and shea butter oil;
- C₆-C₁₈ acid carbonates, such as dioctyl carbonate;
- synthetic esters and ethers, especially of fatty acids, for instance the oils of formulae R₁COOR₂ and R₁OR₂ in which R₁ represents the residue of a fatty acid containing from 8 to 29 carbon atoms and R₂ represents a branched or unbranched hydrocarbon-based chain containing from 3 to 30 carbon atoms, for instance Purcellin oil, isononyl isononanoate, isopropyl myristate, 2-ethylhexyl palmitate, 2-octyldodecyl stearate, 2-octyldodecyl erucate or isostearyl isostearate; hydroxylated esters, for instance isostearyl lactate, octyl hydroxystearate, octyldodecyl hydroxystearate, diisostearyl malate or triisocetyl citrate; fatty alcohol heptanoates, octanoates or decanoates; polyol esters, for instance propylene glycol dioctanoate, neopentyl glycol diheptanoate and diethylene glycol diisononanoate; and pentaerythritol esters, for instance pentaerythrityl tetraisostearate;
- linear or branched hydrocarbons of mineral or synthetic origin, such as volatile or nonvolatile liquid paraffins, and the derivatives thereof, petroleum jelly, polydecenes, hydrogenated polyisobutene, such as Parleam oil, isododecane, isohexadecane, etc.;
- fatty alcohols containing from 8 to 26 carbon atoms, for instance cetyl alcohol, stearyl alcohol and the mixture thereof (cetylstearyl alcohol), octyldodecanol, 2-butyloctanol, 2-hexyldecanol, 2-undecylpentadecanol, oleyl alcohol or linoleyl alcohol;
- partially hydrocarbon-based and/or silicone-based fluoro oils, for instance those described in the document JP-A-2 295 912;
- silicone oils, for instance volatile or nonvolatile polydimethylsiloxanes (PDMS) with a linear or cyclic silicone chain, which are liquid or pasty at room temperature, especially cyclopolydimethylsiloxanes (cyclomethicones) such as cyclohexasiloxane; polydimethylsiloxanes comprising alkyl, alkoxy or phenyl groups, which are pendent or at the end of a silicone chain, these groups containing from 2 to 24 carbon atoms; phenyl silicones, for instance phenyl trimethicones, phenyl dimethicones, phenyltrimethylsiloxydiphenylsiloxanes, diphenyl dimethicones, diphenylmethyldiphenyltrisiloxanes or 2-phenylethyl trimethylsiloxy silicates, and polymethylphenylsiloxanes;
- the mixtures thereof.

In the list of oils mentioned above, the term "hydrocarbon-based oil" is intended to mean any oil mainly comprising carbon and hydrogen atoms, and possibly ester, ether, fluoro, carboxylic acid and/or alcohol groups.

The ratio of fatty alcohol according to the invention a) to oil may vary from 0.05 to 0.5, preferably from 0.1 to 0.3 and even more particularly from 0.15 to 0.25.

The proportion of oil in the emulsion may range from 2% to 50% by weight, preferably from 5% to 20% by weight and even more particularly from 10% to 15% by weight relative to the total weight of the composition.

The other fatty substances that may be present in the oily phase are, for example, fatty acids containing from 8 to 30 carbon atoms, for instance stearic acid, lauric acid, palmitic acid and oleic acid; waxes, for instance lanolin, beeswax, carnauba wax or candelilla wax, paraffin or lignite waxes or microcrystalline waxes, ceresin or ozokerite, and synthetic waxes, for instance polyethylene waxes and Fischer-Tropsch waxes; silicone resins such as trifluoromethyl-C₁-C₄-alkyl dimethicone and trifluoropropyl dimethicone; and silicone elastomers, for instance the products sold under the name KSG by the company Shin-Etsu, under the name Trefil, BY29 or EPSX by the company Dow Corning or under the name Gransil by the company Grant Industries.

These fatty substances may be chosen in a varied manner by a person skilled in the art so as to prepare a composition having the desired properties, for example in terms of consistency or texture.

According to one particular embodiment of the invention, the composition according to the invention is a water-in-oil (W/O) or oil-in-water (O/W) emulsion, preferably an O/W emulsion.

The O/W emulsion also comprises emulsified gels. The term "emulsified gels" is intended to mean dispersions of oils in an aqueous gel. The addition of surfactant is optional for this galenic form.

The proportion of the oily phase of the emulsion may range from 2% to 80% by weight and preferably from 5% to 50% by weight relative to the total weight of the composition.

The emulsions generally contain at least one emulsifier chosen from amphoteric, anionic, cationic or nonionic emulsifiers, used alone or as a mixture, and optionally a coemulsifier. The emulsifiers are chosen in an appropriate manner according to the emulsion to be obtained (W/O or O/W). The emulsifier and the coemulsifier are generally present in the composition in a proportion ranging from 0.3% to 30% by weight and preferably from 0.5% to 20% by weight relative to the total weight of the composition.

The composition may be an aqueous gel, and may especially comprise common aqueous gelling agents.

Advantageously, the composition is a composition comprising at least one aqueous phase. The composition according to the invention preferably comprises an aqueous phase comprising water and a water-soluble organic solvent chosen for example from linear or branched C₂-C₄ alkanols, such as ethanol, isopropanol, propanol or butanol, polyols and polyol ethers, for instance 2-butoxyethanol, glycerol, diglycerol, propylene glycol, dipropylene glycol, butylene glycol, hexylene glycol, 1,3-propanediol, pentylene glycol, polyethylene glycols, propylene glycol monomethyl ether, diethylene glycol monomethyl ether and monoethyl ether, and also aromatic alcohols, for instance benzyl alcohol or phenoxyethanol, and the mixtures thereof.

The composition generally comprises from 30% to 95% by weight of water relative to the total weight of the composition.
The amount of organic solvent(s) can range, for example, from 0 to 30% by weight, preferably from 0.5% to 20% by weight, better still from 1% to 15% by weight, even better still from 2% to 10% by weight and even better still from 2% to 8% by weight relative to the total weight of the composition.

According to one preferred mode, the composition may comprise at least one C₈-C₃₀ alkyl(poly)glycoside. These surfactants may more particularly be represented by the following general formula:

R₁O-(R₂O)ₜ(G)ᵥ

in which R₁ represents a linear or branched alkyl and/or alkenyl radical comprising approximately from 8 to 30 carbon atoms, an alkylphenyl radical, the linear or branched alkyl radical of which comprises from 8 to 24 carbon atoms, R₂ represents an alkylene radical comprising approximately from 2 to 4 carbon atoms, G represents a sugar unit comprising from 5 to 6 carbon atoms, t denotes a value ranging from 0 to 10, preferably from 0 to 4, preferably from 0 to 3, and v denotes a value ranging from 1 to 15, preferably from 1 to 4.

It should also be noted that each unit of the polysaccharide part of the alkylpolyglycoside may be in α or β isomeric form, in L or D form, and the configuration of the saccharide residue may be of furanoside or pyranoside type.

It is, of course, possible to use mixtures of alkylpolysaccharides, which may differ from one another in the nature of the borne alkyl unit and/or the nature of the bearing polysaccharide chain.

According to a specific embodiment, the alkyl(poly)glycoside surfactants are compounds with the formula described above in which R₁ more particularly denotes a saturated or unsaturated and linear or branched alkyl radical comprising from 12 to 24 carbon atoms, t denotes a value ranging from 0 to 3 and more particularly still equal to 0, and G may denote glucose, fructose, galactose, maltose, maltotriose, lactose, cellobiose, mannose, ribose, dextran, talose, allose, xylose, levoglucan, cellulose or starch, preferably glucose. The degree of polymerization, i.e. the value of v in the above formula, may range from 1 to 5, preferably from 1 to 4. The average degree of polymerization is more particularly between 1 and 2.5, preferably from 1.05 to 2.5 and more preferably from 1.1 to 2.
The glucoside bonds between the sugar units are of 1-6 or 1-4 type and preferably of 1-4 type.
It is possible especially to use coco(poly)glucoside (for example Montanov 82^{®} and Montanov S), arachidyl(poly)glucoside (for example Montanov 202), myristyl(poly)glucoside (for example Montanov 14^{®}), cetylstearyl(poly)glucoside (for example Montanov 68^{®}), C₁₂-C₂₀ alkyl(poly)glucosides (for example Montanov L ^{®}), isostearyl(poly)glucoside (for example Montanov WO 18^{®}) or octyldodecyl(poly)xyloside (for example Fluidanov 20X ^{®}).

Preference will be given to arachidyl(poly)glucoside, such as the commercial product Montanov 202^{®} from SEPPIC.

According to a specific embodiment of the invention, the content of alkyl(poly)glycosides varies from 0.05% to 10% by weight, preferably from 0.1 % to 5% by weight, more preferably from 0.2% to 1% by weight and better still from 0.25% to 0.6% by weight relative to the total weight of the composition.

Advantageously, the composition according to the invention has a pH ranging from 3 to 8. Preferably, the pH of the composition ranges from 4 to 7.

The composition according to the invention may be a composition for caring for, cleaning or making up the skin of the body or the face, in particular a care composition.
The composition for caring for the skin may for example be a cream, a gel or a fluid for the face.

### Additives:

The composition according to the present invention may also contain various adjuvants which are commonly used in the field of cosmetics, such as surfactants, emulsifiers, fillers, preserving agents, sequestrants, dyes, fragrances, pH adjusting agents.

Advantageously, the composition may also comprise at least one active agent for caring for oily skin, preferably chosen from the cosmetic active agents such as desquamating agents, antimicrobial agents, anti-inflammatory agents, sebum-regulating agents, antioxidants, moisturizers and vitamins.

Needless to say, those skilled in the art will take care to select this or these optional additional compound(s), and/or the amount thereof, such that the advantageous properties of the composition according to the invention are not, or are not substantially, adversely affected by the envisaged addition.

The active agent(s) used in the composition according to the invention may represent from 0.0001 % to 20%, preferably from 0.001 % to 10% and better still from 0.01 % to 5% of the total weight of the composition.

Another subject of the invention is a cosmetic process for caring for and/or making up the skin, comprising the topical application to the skin of the composition according to the invention.

More specifically, this is a process for mattifying the skin and/or reducing the shininess thereof, comprising the topical application to the skin of the composition according to the invention.
The term "mattify" is intended to mean making the skin more matte, reducing the shininess thereof and therefore the esthetically displeasing reflections thereof.

The invention also relates to the cosmetic use of the composition according to the invention for caring for combination and/or oily skin.

Another subject of the invention is a process for moisturizing the skin, comprising the topical application to the skin of the composition according to the invention.

The invention will now be illustrated with the aid of the nonlimiting examples that follow. In these examples, the amounts are indicated as weight percentages. The compounds are, depending on the case, cited as the chemical names or as the CTFA (International Cosmetic Ingredient Dictionary and Handbook) names.

### EXAMPLES

### Example 1: Measuring the matte finish/shininess

| In % AM | A | B |
|---|---|---|
| **Behenyl behenate** | 3 | - |
| **Cetylstearyl alcohol** | - | 3 |
| Arachidyl Alcohol(55%) (and) Behenyl Alcohol(30%) (and) Arachidyl Glucoside(15%) (Montanov 202 from SEPPIC) | 3 | 3 |
| Isohexadecane | 6 | 6 |
| Dimethicone 10 cSt | 6 | 6 |
| Sodium Polyacryloyldimethyl Taurate in inverse emulsion (Simulgel 800 from SEPPIC) | 0.72 | 0.72 |
| Preserving agents | 0.5 | 0.5 |
| Water q.s. for | 100% | 100% |

### Measuring the matte finish by in vitro evaluation

The matte finish obtained with composition A according to the invention and with composition B given by way of comparative example was measured, using a contrast card (Prufkarte 24/5 - 250 cm² type) sold by the company Erichsen. The composition was spread in an amount of 2 mg/cm² by means of a mechanical film drawer to obtain a film 100 microns thick. The cards are stored for 24 h at 37°C under an atmosphere controlled between 15% and 25% humidity.

1 spray of a mixture (20% oleic acid + 79% Vichy water + 1% Oleth-10) was carried out; the amount deposited is approximately 0.3 g per spray. Reflection was measured using a gonioreflectometer at T0 (GU T0), then there was a 6 minute wait at room temperature and then a new measurement was taken (GU T6 min). The result obtained is the ratio R between the specular reflection and the scattered reflection (gloss unit). The value of R is proportionately smaller the greater the mattifying effect.

The table below gives the values for immediate gloss unit (GU T0) and for after spraying on an artificial sebum/sweat solution (GU T6 min).

| Composition | Ingredient at 3% | Gloss unit GU T0 | GU T6 min |
|---|---|---|---|
| B | Cetylstearyl alcohol | 9 | 45 |
| A | Behenyl behenate (Dub BB from Stéarinerie Dubois) | 7 | 10 |

These results show that the composition according to the invention makes it possible to obtain a lower level of shininess than that obtained with a composition containing a C₁₆-C₁₈ for fatty alcohol.

The performance of the composition is maintained in the presence of sweat and/or sebum.

### Example 2:

The following 3 compositions were prepared:

| In % AM | A | B | c |
|---|---|---|---|
| **Behenyl behenate** | 1 | - | |
| **Glyceryl monobehenate, dibehenate and tribehenate (Compritol 888 CG from Gattefosse)** | - | 1 | |
| **Glyceryl trihydroxystearate (Thixcin R from Elementis)** | | | 1 |
| Arachidyl Alcohol(55%) (and) Behenyl Alcohol(30%) (and) Arachidyl Glucoside(15%) (Montanov 202 from SEPPIC) | 3 | 3 | 3 |
| Isohexadecane | 6 | 6 | 6 |
| Dimethicone 10 cSt | 6 | 6 | 6 |
| Sodium Polyacryloyldimethyl Taurate in inverse emulsion (Simulgel 800 from SEPPIC) | 0.72 | 0.72 | 0.72 |
| Glycerol | 5 | - | - |
| Preserving agents | 0.5 | 0.5 | 0.5 |
| Water q.s. for | 100% | 100% | 100% |

### Measuring the matte finish by in vitro evaluation

The procedure was carried out as described in example 1

The table below gives the values for immediate gloss unit (GU T0) and for gloss unit after spraying on an artificial sebum/sweat solution (GU T6 min).

| Composition | | Gloss unit GU T0 | GU T6 min |
|---|---|---|---|
| A | Behenyl behenate | 2.9 | 4.3 |
| B | Glyceryl tribehenate | 3.25 | 7.2 |
| C | Glyceryl trihydroxystearate | 3.2 | 3.2 |

The performance of the compositions is maintained in the presence of sweat and/or sebum.

### Example 3:

A composition according to the invention was prepared:

| INCI Name | % Active Material |
|---|---|
| Arachidyl Alcohol(55%) (and) Behenyl Alcohol(30%) (and) Arachidyl Glucoside(15%) Montanov 202 from SEPPIC | 3 |
| Isohexadecane | 6 |
| Dimethicone (DC Toray SH 200C fluid 10C5 from Dow Corning) | 6 |
| Behenyl Behenate DUB BB (100%) | 1 |
| Glycerol | 5 |
| Sodium Polyacryloyldimethyl Taurate in inverse emulsion (Simulgel 800 from SEPPIC) | 0.72 |
| Preserving agents | 0.5 |
| Water q.s. for | 100 % |
| | |
| Gloss unit T0 | 2.9 |
| Gloss unit T6 min | 4.3 |

The performance of the composition is maintained in the presence of sweat and/or sebum.

This composition may be applied in the morning and/or evening to the face to mattify and moisturize combination and oily skin.

### Example 4:

| INCI Name | Concentration |
|---|---|
| Behenyl Behenate - DUB BB | 3 |
| Isohexadecane | 6 |
| Dimethicone (DC Toray SH 200C fluid 10C5 from Dow Corning) | 6 |
| Water | q.s. for 100 % |
| Sodium Polyacryloyldimethyl Taurate in inverse emulsion (Simulgel 800 from SEPPIC) | 0.72 |
| Phenoxyethanol | 0.5 |
| | |
| Gloss unit T0 | 7 |
| Gloss unit T6 min | 10 |

The performance of the composition is maintained in the presence of sweat and/or sebum.

This composition may be applied in the morning and/or evening to the face to mattify combination and oily skin.

### Example 5 : Measuring the matte finish/shininess during time, with repeated presence of sweat and/or sebum

| INCI Name | A | B |
|---|---|---|
| Behenyl Behenate - DUB BB | - | 1 |
| Arachidyl Alcohol(55%) (and) Behenyl Alcohol(30%) (and) Arachidyl Glucoside(15%) Montanov 202 from SEPPIC | 3 | 3 |
| ISOHEXADECANE | 6 | 6 |
| DIMETHICONE 10 Cst | 6 | 6 |
| Sodium Polyacryloyldimethyl Taurate in inverse emulsion (Simulgel 800 from SEPPIC) | 2 | 1 |
| Phenoxyethanol | 0,5 | 0,5 |
| water qs for | 100% | 100% |

### Measuring the matte finish by in vitro evaluation

The matte finish obtained with composition B according to the invention and with composition A given by way of comparative example was measured, using a contrast card (Prufkarte 24/5 - 250 cm² type) sold by the company Erichsen. The composition was spread in an amount of 2 mg/cm² by means of a mechanical film drawer to obtain a film 100 microns thick. The cards are stored for 24 h at 37°C under an atmosphere controlled between 15% and 25% humidity.

5 successive sprays of a same mixture reproducing an artificial solution of sweat/sebum (20% oleic acid + 79% Vichy water + 1% Oleth-10) was carried out; the amount deposited is approximately 0.3 g per spray. Reflection was measured using a gonioreflectometer at T0 (GU T0), then there was a 6 minute wait at room temperature after each spraying before new reflection measures. The result obtained is the ratio R between the specular reflection and the scattered reflection (gloss unit). The value of R is proportionately smaller the greater the mattifying effect.

The table below gives the values for immediate gloss unit (GU T0) and for after spraying on an artificial sebum/sweat solution.

| | A | B |
|---|---|---|
| gloss unit (GU) | | |
| Initial gloss unit (GU T0) | 2,7 | 2,8 |
| GU after 1 spray | 5,4 | 3,5 |
| GU after 2 sprays | 21,6 | 9 |
| GU after 3 sprays | 41,3 | 15 |
| GU after 4 sprays | 51 | 17 |
| GU after 5 sprays | 54 | 27 |

The performance of the composition is maintained in the repeated presence of sweat and/or sebum.

This composition may be applied in the morning and/or evening to the face to mattify combination and oily skin, and can maintain its matte finish properties, even when it is subjected to the repeated presence of sweat and/or sebum.

## Claims

1. A composition comprising, in a physiologically acceptable medium:
a) one or more fatty alcohols comprising at least 20 carbon atoms,
b) one or more fatty acid esters, said fatty acid ester is behenyl behenate; and
c) at least one or more thickeners which are not xanthan gum, wherein the thickeners are chosen from homopolymers and copolymers based on acrylic acid or methacrylic acid which may or may not be salified, homopolymers of 2-acrylamido-2-methylpropanesulfonic acid which may or may not be salified, and/or cellulose polymers, and anionic associative thickeners, preferably acrylic, which may or may not be salified. and
d) at least one C₈-C₃₀ alkyl(poly)glycoside

2. The composition as claimed in claim 1, the fatty alcohol(s) comprising at least 20 carbon atoms are of structure R^{a-}OH, in which R^{a} denotes a saturated or unsaturated, linear or branched, radical comprising from 20 to 50 carbon atoms, preferably from 20 to 30 carbon atoms; R^{a} may be substituted with one or more hydroxyl groups.

3. The composition as claimed in either one of the preceding claims, **characterized in that** the fatty alcohol(s) comprising at least 20 carbon atoms are primary fatty alcohols which have a saturated linear backbone, such as especially behenyl alcohol, arachidyl alcohol, lignoceryl alcohol, ceryl alcohol and montanyl alcohol, and more particularly behenyl alcohol and arachidyl alcohol.

4. The composition as claimed in any one of the preceding claims, **characterized in that** the amount of fatty alcohol(s) comprising at least 20 carbon atoms varies from 0.1% to 20% by weight, particularly from 0.2% to 10% by weight and preferably from 0.5% to 5% by weight relative to the total weight of the composition.

5. The composition as claimed in any one of the preceding claims, **characterized in that** said fatty acid esters are present in the compositions according to the invention in concentrations ranging from 0.1% to 10% by weight and preferably from 0.5% to 5% by weight relative to the total weight of the composition.

6. The composition as claimed in any one of claims 1 to 5, **characterized in that** the thickener(s) according to the invention are present in a content ranging from 0.01% to 20% by weight, preferably ranging from 0.1% to 20% by weight and most preferably ranging from 0.5% to 10% by weight relative to the total weight of the composition.

7. The composition as claimed in claim 1, **characterized in that** the C₈-C₃₀ alkyl(poly)glycoside(s) are present in a content ranging from 0.05% to 10% by weight, preferably from 0.1% to 5% by weight, more preferably from 0.2% to 1% by weight and better still from 0.25% to 0.6% by weight relative to the total weight of the composition.

8. The composition as claimed in any one of the preceding claims, **characterized in that** it also comprises at least one oil.

9. The composition as claimed in any one of the preceding claims, **characterized in that** it also comprises at least one active agent for caring for oily skin, chosen from desquamating agents, antimicrobial agents, anti-inflammatory agents, sebum-regulating agents and antioxidants.

10. A cosmetic process for caring for and/or making up the skin, comprising the topical application to the skin of the composition as claimed in one of claims 1 to 9.

11. The process as claimed in claim 10, **characterized in that** it is a process for mattifying the skin and/or reducing the shininess thereof.

12. The cosmetic use of the composition as claimed in any one of claims 1 to 9, for caring for combination and/or oily skin.

13. The use of an acid ester as claimed in one of claims 1 and 5, as agent for mattifying keratin materials such as oily and/or shiny skin.

## Patentansprüche

1. Zusammensetzung, umfassend in einem physiologisch verträglichen Medium:
a) einen oder mehrere Fettalkohole, die wenigstens 20 Kohlenstoffatome umfassen,
b) einen oder mehrere Fettsäureester, wobei der Fettsäureester Behenylbehenat ist; und
c) wenigstens ein oder mehrere Verdickungsmittel, die nicht Xanthangummi sind, wobei die Verdickungsmittel ausgewählt sind aus Homopolymeren und Copolymeren auf der Grundlage von Acrylsäure oder Methacrylsäure, die versalzt sein können oder auch nicht, Homopolymeren von 2-Acrylamido-2-methylpropansulfonsäure, die versalzt sein können oder auch nicht, und/oder Cellulosepolymeren, und anionischen assoziativen Verdickungsmitteln, vorzugsweise acrylischen, die versalzt sein können oder auch nicht, und
d) wenigstens ein C₈-C₃₀Alkyl(poly)glycosid.

2. Zusammensetzung gemäß Anspruch 1, wobei die Fettalkohol(e), die wenigstens 20 Kohlenstoffatome umfassen, von der Struktur **R^{a}-OH** sind, wobei **R^{a}** einen gesättigten oder ungesättigten, linearen oder verzweigten Rest bezeichnet, der von 20 bis 50 Kohlenstoffatome, vorzugsweise von 20 bis 30 Kohlenstoffatome, umfasst; wobei **R^{a}** mit einer oder mehreren Hydroxygruppen substituiert sein kann.

3. Zusammensetzung gemäß einem der vorstehenden Ansprüche, **dadurch gekennzeichnet, dass** die Fettalkohol(e), die wenigstens 20 Kohlenstoffatome umfassen, primäre Fettalkohole sind, die ein gesättigtes lineares Gerüst aufweisen, wie z. B. insbesondere Behenylalkohol, Arachidylaklohol, Lignocerylalkohol, Cerylalkohol und Montanylalkohol, besonders Behenylalkohol und Arachidylaklohol.

4. Zusammensetzung gemäß einem der vorstehenden Ansprüche, **dadurch gekennzeichnet, dass** die Menge an Fettalkohol(en), die wenigstens 20 Kohlenstoffatome umfassen, von 0,1 Gew.-% bis 20 Gew.-%, insbesondere von 0,2 Gew.-% bis 10 Gew.-% und vorzugsweise von 0,5 Gew.-% bis 5 Gew.-%, bezogen auf das Gesamtgewicht der Zusammensetzung, variiert.

5. Zusammensetzung gemäß einem der vorstehenden Ansprüche, **dadurch gekennzeichnet, dass** die Fettsäureester in den Zusammensetzungen gemäß der Erfindung in Konzentrationen in dem Bereich von 0,1 Gew.-% bis 10 Gew.-% und vorzugsweise von 0,5 Gew.-% bis 5 Gew.-%, bezogen auf das Gesamtgewicht der Zusammensetzung, vorhanden sind.

6. Zusammensetzung gemäß einem der Ansprüche 1 bis 5, **dadurch gekennzeichnet, dass** das/die Verdickungsmittel gemäß der Erfindung in einem Gehalt in dem Bereich von 0,01 Gew.-% bis 20 Gew.-%, vorzugsweise in dem Bereich von 0,1 Gew.-% bis 20 Gew.-% und höchst bevorzugt in dem Bereich von 0,5 Gew.-% bis 10 Gew.-%, bezogen auf das Gesamtgewicht der Zusammensetzung, vorhanden sind.

7. Zusammensetzung gemäß Anspruch 1, **dadurch gekennzeichnet, dass** die C₈-C₃₀Alkyl(poly)glycosid(e) in einem Gehalt in dem Bereich von 0,05 Gew.-% bis 10 Gew.-%, vorzugsweise von 0,1 Gew.-% bis 5 Gew.-%, bevorzugter von 0,2 Gew.-% bis 1 Gew.-% und noch besser von 0,25 Gew.-% bis 0,6 Gew.-%, bezogen auf das Gesamtgewicht der Zusammensetzung, vorhanden sind.

8. Zusammensetzung gemäß einem der vorstehenden Ansprüche, **dadurch gekennzeichnet, dass** sie ferner wenigstens ein Öl umfasst.

9. Zusammensetzung gemäß einem der vorstehenden Ansprüche, **dadurch gekennzeichnet, dass** sie ferner wenigstens einen Wirkstoff zur Pflege von öliger Haut ausgewählt aus abschuppenden Mitteln, antimikrobiellen Mitteln, entzündungshemmenden Mitteln, sebumregulierenden Mitteln und Antioxidationsmitteln umfasst.

10. Kosmetisches Verfahren zur Pflege und/oder zum Makeup der Haut, umfassend das topische Aufbringen der Zusammensetzung gemäß einem der Ansprüche 1 bis 9 auf die Haut.

11. Verfahren gemäß Anspruch 10, **dadurch gekennzeichnet, dass** es ein Verfahren zum Mattieren der Haut und/oder zum Verringern ihres Glanzes ist.

12. Kosmetische Verwendung der Zusammensetzung gemäß einem der Ansprüche 1 bis 9 zur Pflege von Mischhaut und/oder öliger Haut.

13. Verwendung eines Säureesters gemäß einem der Ansprüche 1 und 5 als Mittel zum Mattieren von Keratinmaterialien, wie z. B. öliger und/oder glänzender Haut.

## Revendications

1. Composition comprenant, dans un milieu physiologiquement acceptable :
a) un ou plusieurs alcools gras comprenant au moins 20 atomes de carbone,
b) un ou plusieurs esters d'acide gras, ledit ester d'acide gras est le béhénate de béhényle ; et
c) au moins un ou plusieurs épaississants qui ne sont pas de la gomme xanthane, les épaississants étant choisis parmi des homopolymères et des copolymères à base d'acide acrylique ou d'acide méthacrylique qui peuvent être ou ne pas être salifiés, des homopolymères d'acide 2-acrylamido-2-méthylpropanesulfonique qui peuvent être ou ne pas être salifiés, et/ou des polymères de cellulose, et des épaississants associatifs anioniques, préférablement acryliques, qui peuvent être ou ne pas être salifiés, et
d) au moins un C₈-C₃₀-alkyl(poly)glycoside.

2. Composition selon la revendication 1, le ou les alcools gras comprenant au moins 20 atomes de carbone qui sont de structure **R^{a-}OH,** dans laquelle **R^{a}** désigne un radical saturé ou insaturé, linéaire ou ramifié, comportant de 20 à 50 atomes de carbone, de préférence de 20 à 30 atomes de carbone ; **R^{a}** peut être substitué par un ou plusieurs groupements hydroxyle.

3. Composition selon l'une quelconque des revendications précédentes, **caractérisée en ce que** le ou les alcools gras comprenant au moins 20 atomes de carbone sont des alcools gras primaires qui présentent une chaîne linéaire saturée tels que notamment l'alcool béhénylique, l'alcool arachidylique, l'alcool lignocérylique, l'alcool cérylique et l'alcool montanylique et plus particulèrement l'alcool béhénylique et l'alcool arachidylique.

4. Composition selon l'une quelconque des revendications précédentes, **caractérisée en ce que** la quantité en alcool(s) gras comprenant au moins 20 atomes de carbone varie de 0,1 à 20 % en poids, particulièrement de 0,2 à 10 % en poids, de préférence de 0,5 à 5 % en poids, par rapport au poids total de la composition.

5. Composition selon l'une quelconque des revendications précédentes, **caractérisée en ce que** lesdits esters d'acides gras sont présents dans les compositions selon l'invention dans des concentrations allant de 0,1 à 10 % en poids et de préférence de 0,5 à 5 % en poids par rapport au poids total de la composition.

6. Composition selon l'une quelconque des revendications 1 à 5, **caractérisée en ce que** le ou les épaississants selon l'invention sont présents en une teneur allant de 0,01 à 20 % en poids, de préférence de 0,1 à 20 % en poids, et plus préférentiellement allant de 0,5 à 10 % en poids, par rapport au poids total de la composition.

7. Composition selon la revendication 1, **caractérisée en ce que** le ou les C₈-C₃₀-alkyl (poly)glycoside(s) sont présents en une teneur allant de 0,05 à 10 % en poids, de préférence de 0,1 à 5 % en poids, et de manière plus préférée de 0,2 à 1 % en poids, mieux de 0,25 à 0,6 % en poids, par rapport au poids total de la composition.

8. Composition selon l'une quelconque des revendications précédentes, **caractérisée en ce qu'**elle comprend en outre au moins une huile.

9. Composition selon l'une quelconque des revendications précédentes, **caractérisée en ce qu'**elle comprend en outre au moins un agent actif de soin des peaux grasses choisi parmi les agents desquamants, anti-microbiens, antiinflammatoires, sébo-régulateurs et anti-oxydants.

10. Procédé cosmétique de soin et/ou de maquillage de la peau, comprenant l'application topique sur la peau de la composition selon l'une des revendications 1 à 9.

11. Procédé selon la revendication 10, **caractérisé en ce qu'**il s'agit d'un procédé pour matifier la peau et/ou réduire sa brillance.

12. Utilisation cosmétique de la composition selon l'une quelconque des revendications 1 à 9, pour le soin des peaux mixtes et/ou grasses.

13. Utilisation d'un ester d'acide selon l'une des revendications 1 et 5 comme agent pour matifier les matières kératiniques telles que la peau grasse et/ou luisante.
